(19) Europäisches Patentamt
European Patent Office
Office européen des brevets

(11) Publication number : **0 037 256**
**B1**

## (12) EUROPEAN PATENT SPECIFICATION

(45) Date of publication of patent specification : 14.09.83

(51) Int. Cl.³ : **C 07 C103/52**

(21) Application number : 81301306.7

(22) Date of filing : 26.03.81

(54) Process for producing an insulin.

(30) Priority : 27.03.80 US 134390

(43) Date of publication of application : 07.10.81 Bulletin 81/40

(45) Publication of the grant of the patent : 14.09.83 Bulletin 83/37

(84) Designated contracting states : AT BE CH DE FR GB IT LI LU NL SE

(56) References cited :
NATURE, vol. 188, n° 4752, November 26, 1960, G.H. DIXON et al. : « Regeneration of Insulin Activity from the Separated and Inactive A and B chains », pages 721-724

TETRAHEDRON LETTERS, n° 12, 1973, Pergamon Press, Great Britain, S.M.L. ROBINSON et al. : «Spaltung und Rückbildung der Disulfid-Brücken an intramolekular vernetzten Insulinen », pages 985-988

(73) Proprietor : **ELI LILLY AND COMPANY**
**307, East McCarty Street**
**Indianapolis Indiana 46285 (US)**

(72) Inventor : **Chance, Ronald Eugene**
**19303 Flippin Road**
**Westfield Indiana (US)**
Inventor : **Hoffmann, James Arthur**
**7404 Earl Court**
**Indianapolis Indiana (US)**

(74) Representative : **Crowther, Terence Roger et al**
**Erl Wood Manor**
**Windlesham Surrey GU20 6PH (GB)**

# Process for producing an insulin

With the advent of the possibility to generate protein products by recombinant DNA methods and specifically the production of insulin A-chain and insulin B-chain by such techniques [see Goeddel *et al.*, *Proc. Nat'l. Acad. Sci. USA 76*, 106-110 (1979)], the need for an efficient method for combining the A- and B-chains to form insulin has greatly increased.

Typically, the prior art methods for producing insulin by combination of A- and B-chains use as starting materials such A- and B-chains in the form of their stable S-sulfonates. In general, the A- and B-chain S-sulfonates, either separately or together, are reduced to their corresponding —SH compounds, customarily using a large excess of thiol reducing agent. The products are isolated from the reducing medium and, if not reduced together, are brought together in an oxidizing medium, e.g., air, to achieve combination of A- and B-chains with production of insulin. Examples of this methodology are found in Du *et al.*, *Scientia Sinica 10*, 84-104 (1961) ; Wilson *et al.*, *Biochim. Biophys. Acta 62*, 483-489 (1962) ; Du *et al.*, *Scientia Sinica 14*, 229-236 (1965) ; Kung *et al.*, *Scientia Sinica 15*, 544-561 (1966) ; *Kexue Tongbao* (Republic of China) *17*, 241-277 (1966) ; and Markussen, *J. Acta Paediatrica Scandinavica, Suppl. 270*, 121-126 (1977).

A modification of this approach involves reduction of the A-chain S-sulfonate followed by reaction of the reduced A-chain with B-chain S-sulfonate in an oxidizing atmosphere. See, e.g., Katsoyannis *et al.*, *Proc. Nat. Acad. Sci. (U.S.A.) 55*, 1554-1561 (1966) ; Katsoyannis, *Science 154*, 1509-1514, (1966) ; Katsoyannis *et al.*, *Biochemistry 6*, 2642-2655 (1967) ; U.S. Patent No. 3, 420, 810 ; and Jentsch, *Journal of Chromatography 76*, 167-174 (1973).

Another modification involves partial oxidation of the A-chain —SH compound to produce disulfide formation between the A-6 and A-11 cysteine residues followed by oxidation of the product with B-chain —SH or B-chain S-sulfonate. See, e.g., Belgian Patent No. 676,069 and Zahn *et al.*, *Liebigs Ann. Chem. 691*, 225-231, (1966).

In each of the above prior art methods, one element is common i.e., the production of insulin by two independent, sequential steps, namely, reduction of S-sulfonate to —SH followed by oxidation to —S—S—.

Dixon *et al.*, *Nature 188*, 721-724 (1960) describe conditions which suggest single solution conversion of A- and B-chain S-sulfonates to insulin using a thiol reducing agent and air oxidation. The details are quite sketchy, and the yield, based only on activity of product recovered, represented 1-2 %. However, Dixon, in *Proc. Intern. Congr. Endocrinol.* 2nd London 1964, 1207-1215 (1965), in somewhat further elaboration, suggests, in Table IV at page 1211, that the conditions reported in the earlier publication involve separate reduction and oxidation steps.

In distinction to the above prior art methods, it has now been discovered that it is possible under defined reaction conditions to achieve attractive levels of production of insulins or analogs of insulins from S-sulfonated A- and B-chains by conducting both the reduction and oxidation reactions in a single-step, single-solution process. It is to such a process that this invention is directed.

Therefore, this invention is directed to a process for combining an A-chain of an insulin or insulin analog and a B-chain of an insulin or an insulin analog to produce an insulin or an insulin analog, which comprises reacting the S-sulfonated form of the A-chain, the S-sulfonated form of the B-chain, and a thiol reducing agent together in an aqueous medium under conditions which produce a mixture having (1) a pH of from 8 to 12, (2) a total protein concentration of from 0.1 to 50 milligrams per milliliter, and (3) an amount of thiol reducing agent which affords a total of from 0.4 to 2.5 —SH groups per each —$SSO_3^-$ group present in the total amount of A- and B-chain S-sulfonates ; and allowing formation of insulin or an insulin analog to occur by maintaining the mixture at a temperature of from 0 °C to 25 °C and in an environment which provides a source of oxygen.

This invention is directed to an efficient, single-step, single-solution process for producing insulin or an analog of insulin from its constituent S-sulfonated A- and B-chains.

By the term « insulin » is meant, of course, any of the naturally occurring insulins, such as human, bovine, porcine, sheep, fish and avian, as well as a hybrid formed from a combination of an A-chain of one species and a B-chain of another.

By the therm « insulin analog » is meant any of a wide variety of proteins, each of which has the basic A-chain and B-chain containing all of the half-cystine residues in a sequential alignment consistent with that of the natural insulins. The analogs differ from natural insulins by substitution, addition, deletion, or modification of one or more amino acid residues, but with retention of the disulfide bond arrangement and at least a portion of the insulin-like activity. Examples of such analogs are [N-formyl-Gly[1]-A]insulin, Desamino-A[1]-insulin, [Sarcosine[1]-A]insulin, [L-Alanine-[1]-A]insulin, [D-Alanine[1]-A]insulin, [Isoasparagine[21]-A]insulin, [D-Asparagine[21]-A]insulin, [Arginine[21]-A]insulin, [Asparagineamide[21]-A]insulin, [Sarcosine[1]-A, Asparagine[21]-A]insulin, [Norleucine[2]-A]insulin, [Threonine[5]-A]insulin, [Leucine[5]-A]insulin, [Phenylalanine[19]-A]insulin, [D-Tyrosine[19]-A]insulin, [Tyrosine[18]-A, Asparagine[19]A, Arginine[21]-A]insulin, Des[B[28-30]-tripeptide]insulin, Des[B[27-30]-tetrapeptide]insulin, Des[B[26-30]-pentapeptide]insulin, Des[B[27-30]-tetrapeptide, Tyrosinamide-[26]-B]insulin, Des[B[26-30]-pentapeptide, Phenylalaninamide[25]-B]insulin, Des[B[1-4]-tetrapeptide]insulin, Des[B[1-5]-pentapeptide]insulin, [Lysine[22]-

B]insulin, [Leucine⁹-B]insulin, [Leucine¹⁰-B]insulin, Des[Phenylalanine¹-B]insulin, and the like. These and others are described in the literature ; see, for example, Blundell, T., *et al., Advances in Protein Chemistry, 26,* 330-362, Academic Press, N.Y., N.Y. (1972) ; Katsoyannis, P.G., *Treatment of Early Diabetes,* 319-327, Plenum Publishing Corp. (1979) ; Geiger, R., *Chemiker Zeitung,* Reprint 100, 111-129, Dr. A. Hüthig, Publisher, Heidelberg, W. Germany (1976) ; Brandenburg, D. *et al., Biochem J. 125,* 51-52 (1971).

Although the process of this invention is broadly applicable to the production of insulins and insulin analogs, it is highly preferred to use it in the production of naturally-occurring insulins, in particular, human, bovine, or porcine insulins, and most particularly, human insulin.

In carrying out the process of this invention, the combination of A- and B-chain to form insulin or an insulin analog can be achieved over a wide range of ratios of one chain relative to the other. The combination, of course, is inherently limlited by the chain, whether A or B, present in the lesser quantity. In any event, although not essential, the customary ratio of A-chain to B-chain, on a weight basis, is from 0.1 : 1 to 10 : 1. It is highly preferred to carry out the process of this invention using a weight ratio of A-chain to B-chain in the range from 1 : 1 to 3 : 1. It has also been discovered, within this preferred range, that certain ranges are especially advantageous for production of a particular species of insulin. Thus, in the combination of A- and B-chain to produce bovine insulin, it is preferred that the ratio of A-chain to B-chain be within the range of from 1.4 : 1.0 to 1.8 : 1.0. As to porcine insulin the preferred range is from 1.0 : 1.0 to 1.4 : 1.0. As to human insulin, the preferred range is from 1.8 : 1.0 to 2.2 : 1.0.

Another parameter which is significant for carrying out the process of this invention at an optimal level is the protein concentration in the reaction medium. The process can be successfully carried out over a wide range of protein concentrations. Generally, however, the protein concentration will range from 0.1 to 50 mg per ml of reaction medium. Preferably, the protein concentration will be in the range of from 2 to 20 mg per ml Again, it has been discovered, within this latter range, that the optimal protein concentration varies depending upon the species of insulin which is produced. Therefore, as to porcine insulin, it is preferred that the protein concentration range be from 8 to 16 mg per ml, whereas, in the production of human or bovine insulin, the preferred range is from 3 to 8 mg per ml.

The process of this invention is carried out in an aqueous medium. The pH of the medium measured at room temperature generally will range from 8 to 12. Preferably, it will be from 9.5 to 11.0 and optimally will be maintained within the range of from 10.4 to 10.6. The pH of the medium may be maintained in the desired range by addition of a suitable buffering agent. Typical such buffering agents are, for example, glycine, glycyl-glycine, carbonate, tris(hydroxymethyl) aminomethane, N,N-bis(2-hydroxyethyl) glycine, pyrophosphate and N-tris-(hydroxymethyl) methyl-3-aminopropanesulfonic acid which effect pH control within the aforedescribed range. The common and preferred buffering agent is glycine.

The concentration of buffering agent generally ranges from 0.001 M to 2 M. Preferably, the concentration is from 0.01 M to 1 M, and, most preferably, from 0.01 M to 0.1 M.

The A- and B-chains are brought together in the appropriate aqueous medium in the presence of a thiol reducing agent. By « thiol reducing agent » is meant a compound that contains at least one —SH group and has the capacity to effect reduction of the S-sulfonate groups of the A- and B-chains. Although any agent having these characteristics can be employed, a much preferred thiol reducing agent is one which, in its oxidized form, has been cyclized to a highly stable compound. The thiol reducing agent is present in an amount which affords a total of from 0.4 to 2.5 —SH groups per each —$SSO_3^-$ group present in the total amount of A- and B-chain, and, preferably, from 0.9 to 1.1 —SH groups per each $SSO_3^-$ group.

Examples of typical thiol reducing agents are dithiothreitol (DTT), dithioerythritol (DTE), 2-mercaptoethanol, methyl thioglycolate, 3-mercapto-1,2-propanediol, 3-mercaptopropionic acid and thioglycolic acid. Preferred thiol reducing agents are dithiothreitol and dithioerythritol, with dithiothreitol being most preferred.

One of the essential conditions of the process of this invention is that it be carried out in an environment that provides a source of oxygen. This condition can be met merely by permitting the reaction mixture to be open to the air. Although a more direct contact method may be employed, such as, for example, by bubbling air or oxygen into and through the reaction medium, such is not necessary.

In general, therefore, the process of this invention is carried out by preparing a mixture of the A-chain S-sulfonate, the B-chain S-sulfonate, and the thiol reducing agent at desired concentrations in an aqueous medium at a pH of from 8 to 12. The mixture, open to air contact, is gently agitated for a period sufficient to allow chain combination to occur, generally at least 30 minutes. During this period of agitation, the mixture generally is maintained at a temperature of from 0 °C to 25 °C ; preferably, however, the mixture is subjected to moderate cooling to maintain the temperature at the lower end of this range, generally from 2 °C to 10 °C.

Once the reaction period has been completed, the insulin or insulin analog product can be isolated by any of a wide variety of methods, all of which are recognized in the field of insulin technology. The most commonly employed techniques for insulin purification are chromatographic techniques. These are readily applicable in recovering insulin from the process of this invention. These can include gel filtration and ion-exchange chromatography.

Moreover, the product can be assayed for purity and activity by recognized methods such as polyacrylamide gel electrophoresis, amino acid analysis, insulin radioreceptorassay, insulin radioimmunoassay, high performance liquid chromatography (HPLC), ultraviolet spectrum, dansylation and rabbit blood glucose assay.

The insulins which are available from the process of this invention include hybrids comprising the insulin A-chain of one species and the insulin B-chain of another species. The thrust of the process of this invention is directed to a proper combining of A- and B-chain S-sulfonates, and the particular structure of these chains, as long as they truly represent insulin or insulin analog A- and B-chains, is immaterial to the process of this invention.

Although an insulin analog or an insulin hybrid, i. e., an A-chain of one species and a B-chain of another species, can be prepared by the process of this invention, it, of course, is preferred to produce an insulin which is structurally identical to that of a naturally occurring insulin by using an A-chain S-sulfonate and a B-chain S-sulfonate, each of which has the amino acid sequence represented by such insulin. Moreover, it is highly preferred to use the process of this invention to produce porcine, bovine, or human insulin, and most preferably, to produce human insulin.

The insulin or insulin analog A- and B-chains, as already indicated, are available by recombinant DNA methodology. They also can be prepared from natural insulins and by classical peptide synthesis methodology, including either solution or solid phase techniques.

The A- and B-chains are maintained in stable form as S-sulfonates. The S-sulfonate starting materials are available by oxidative sulfitolysis, a treatment by which the -A and B-chains are reacted with sodium sulfite in the presence of a mild oxidizing agent, such as sodium tetrathionate.

As illustrative of the process of this invention, the following examples are provided. These examples are provided for illustrative purposes only, and they are not intended to be limiting upon the scope of this invention.

## Example 1

Porcine A-chain S-sulfonate (360 mg) was dissolved in 36 ml of 0.1 M glycine buffer (pH 10.5), and the pH of the mixture was adjusted to 10.5 with 5 N NaOH. Porcine B-chain S-sulfonate (300 mg) was dissolved in 30 ml of 0.1 M glycine buffer (pH 10.5), and the pH of the mixture was adjusted to 10.5 with 5 N NaOH. Dithiothreitol (DTT) (123.4 mg) was dissolved in 4 ml of the 0.1 M glycine buffer (pH 10.5), and the pH of the mixture was adjusted to 10.5 with 0.2 ml of 5 N NaOH.

The A- and B-chain solutions were combined in a 100 ml vial at room temperature ($\sim$25 °C), and 1.91 ml of the DTT solution then were added to provide an —ST to —$SSO_3^-$ ratio of 1.04. The resulting solution was mixed gently in an open beaker with magnetic stirring at 4-8 °C for 20 hours. Analysis by high performance liquid chromatography (HPLC) indicated an insulin yield of 193.8 mg, or 29 % of the total protein.

Forty ml. of this final solution were adjusted to pH 3.15 with acetic acid. The mixture was gel filtered on a 5 × 200 cm. column of Sephadex® G-50 (Superfine) equilibrated and eluted with 1 M acetic acid at 4-8 °C The insulin peak (elution volume, about 2 450-2 700 ml) was pooled and lyophilized with a recovery of 95 mg of insulin, or 25 % of the total protein. The porcine insulin was judged to be quite pure by polyacrylamide gel electrophoresis, amino acid analysis, insulin radioreceptor assay, HPLC, and the rabbit blood glucose reduction test.

## Example 2

Solutions of bovine A- and B-chain S-sulfonates having a concentration of 5 mg/ml in 0.01 M glycine buffer (pH 10.5) were prepared. The pH of each was adjusted to 10.5 with 5 N NaOH. DTT (61.7 mg) was dissolved in 4.0 ml of 0.1 M glycine buffer (pH 10.5), and the pH was adjusted to 10.5 with 0.15 ml of 5 N NaOH. To 0.5 ml of the B-chain solution were added 0.8 ml of the A-chain solution and 0.035 ml of the DTT solution at room temperature ($\sim$ 25 °C) to provide an —SH to —$SSO_3^-$ ratio of 0.91. The resulting solution was stirred at 4-8 °C for 20 hours in an open 3-ml vial. HPLC analysis of the mixture indicated a bovine insulin yield of 1.96 mg, or 30 % of the total protein.

## Example 3

Solutions of porcine A- and B-chain S-sulfonates having a concentration of 10 mg/ml in 0.1 M glycine buffer (pH 10.5) were prepared. The pH of each was adjusted to 10.5 with 5 N NaOH. DTT (61.7 mg) was dissolved in 2.0 ml of glass-distilled water. To 0.5 ml of the B-chain solution were added 0.6 ml of the A-chain solution and 29.25 μl of the DTT solution at room temperature ($\sim$ 25 °C) to provide an —SH to —$SSO_3^-$ ratio of 1.00. The resulting solution was stirred at 4-8 °C in an open 3-ml vial for 20 hours. HPLC analysis of the mixture indicated a porcine insulin yield of 3.81 mg, or 35 % of the total protein.

## Example 4

Human (pancreatic) B-chain S-sulfonate and several human (pancreatic and *E. coli*) and porcine (pancreatic) A-chain S-sulfonate solutions having a concentration of 5 mg/ml in 0.1 M glycine buffer (pH 10.5) were prepared. The pH of each was adjusted to 10.5 with 5 N NaOH. DTT (61.7 mg) was dissolved in 4.0 ml of 0.1 M glycine buffer (pH 10.5), and the pH was adjusted to 10.5 with 0.16 ml of 5 N NaOH. To 1.0 ml of each of the A-chain S-sulfonate solutions were added 0.5 ml of the B-chain S-sulfonate solution and 0.05 ml of the DTT solution at room temperature (25 °C) to provide an —SH to —SSO$_3^-$ ratio of 1.09. All solutions were stirred in a chill room (4-8 °C) in an open vial for 20-22 hours. They then were analyzed by HPLC using a pancreatic human insulin standard for the yield calculations. The results are in the Table following :

Table 1

| A-Chain Source | Human Insulin Yield, mg. | % Yield Relative to Total Protein |
|---|---|---|
| Porcine (Pancreatic) | 2.00 | 26.7 |
| Porcine (Pancreatic) | 2.11 | 28.1 |
| Human (Pancreatic) | 1.95 | 26.0 |
| Human (Pancreatic) | 2.03 | 27.1 |
| Human (E. coli) | 1.99 | 26.5 |

Example 5

A solution of each of human A- and B-chain S-sulfonates was prepared at a concentration of 5 mg/ml in a 0.1 M glycine buffer (pH 10.5). The pH of each was adjusted to 10.5 with 5 N NaOH. DTT (61.7 mg) was dissolved in 4.0 ml of 0.1 M glycine buffer (pH 10.5), and the pH was adjusted to 10.5 with 0.16 ml of 5 N NaOH. To 0.5 ml of the B-chain solution was added at room temperature 1.0 ml of the A-chain solution followed by 50 µl of the DTT solution to provide an —SH to —SSO$_3^-$ ratio of 1.09. The resulting solution was stirred at 4-8 °C in an open 3-ml vial for 22 hours after which HPLC analysis indicated a human insulin yield of 2.58 mg, or 34 % of the total protein.

Example 6

Human A-chain S-sulfonate (328 mg) was dissolved in 65.6 ml of 0.1 M glycine buffer (pH 10.5), and the pH of the mixture was adjusted to 10.5 with 75 µl of 5 N NaOH. Human B-chain S-sulfonate (164 mg) was dissolved in 32.8 ml of 0.1 M glycine buffer (pH 10.5), and the pH of the mixture was adjusted to 10.5 with 15 µl of 5 N NaOH. DTT (61.7 mg) was dissolved in 4.0 ml of the 0.1 M glycine buffer (pH 10.5), and the pH of the solution was adjusted to 10.5 with 160 µl of 5 N NaOH.

The A- and B-chain solutions were combined in a 150 ml glass beaker at room temperature (~ 25 °C), and 3.28 ml of the DTT solution were added to provide an —SH to —SSO$_3^-$ ratio of 1.09. The open beaker was placed in an ice-water bath in the chill room and stirred briskly for 30 minutes. The solution was stirred an additional 24 hours in the chill room (4-8 °C). HPLC analysis at this time indicated a human insulin yield of 148 mg, or 30 % of the total protein.

To 100 ml of this solution were added 25 ml of glacial acetic acid to a final pH of 3.15. The entire sample was gel filtered on a 5 × 200 cm. column of Sephadex G-50 (Superfine) equilibrated and eluted with 1 M acetic acid at 4-8 °C. All of the eluted protein was lyophilized. The insulin peak (elution volume 2465-2781 ml) weighed 125 mg and represented 29.4 % of the recovered protein.

A portion of the above insulin peak (95.5 mg) was dissolved in about 9 ml of 0.01 M tris-0.001 M EDTA-7.5 M urea-0.03 M NaCl buffer (pH 8.5 at 4 °C). The mixture was chromatographed through a 2.5 × 90 cm. DEAE (diethylaminoethyl)-cellulose ion-exchange column equilibrated in the same buffer. The protein was eluted at 4-8 °C with a gradient of 1 liter each of 0.03 M and 0.09 M NaCl in the same buffer followed by 1 liter of buffer containing 1 M NaCl. Each of the peaks was desalted on Sephadex G-25 (course) columns in 2 % acetic acid and lyophilized. The insulin peak (elution volume 878-1 008 ml) weighed 55.73 mg and represented 84 % of the protein recovered.

Zinc insulin crystals were made by dissolving 11.90 mg of the insulin (DEAE) peak sample in 240 µl of 0.1 N HCl followed quickly by 2.16 ml of a 0.04 % ZnCl$_2$-0.05 M sodium citrate-15 % acetone solution in a glass centrifuge tube. Crystallization proceeded for 72 hours at room temperature (~ 25 °C) after which the supernatant was removed, and the crystals were washed twice with cold pH 6.1 water with centrifugation at 2 000 rpm at 3 °C between washes. The crystals were redissolved in 0.01 N HCl for analysis.

The resulting human insulin preparation was judged to be quite pure by polyacrylamide gel

electrophoresis (a single band), amino acid analysis, insulin radioreceptorassay, insulin radioimmunoassay, HPLC, dansylation and UV spectrum. The USP rabbit assay (144 rabbits) gave a potency of $26.3 \pm 1.8$ units per mg (anhydrous).

Example 7

Human (E. coli) [N-formyl-Gly[1]] A-chain S-sulfonate and human (pancreatic) B-chain S-sulfonate solutions having a concentration of 5 mg/ml in 0.1 M glycine buffer (pH 10.5) were prepared. The pH of each was adjusted to 10.5 with 5 N NaOH. DTT (61.7 mg) was dissolved in 4.0 ml of 0.1 M glycine buffer (pH 10.5), and the pH was adjusted to 10.5 with 0.16 ml of 5 N NaOH. To 0.5 ml of the B-chain S-sulfonate solution were added 1.0 ml of the [N-formyl-Gly[1]] A-chain S-sulfonate solution and 0.05 ml of the DTT solution at room temperature (25 °C) to provide an —SH to —SSO$_3$ ratio of 1.10. The solution was stirred in a chill room (4-8 °C) in an open 3-ml vial for 23 hours after which HPLC analysis indicated a [N-formyl-Gly[1]-A] human insulin yield of 1.46 mg, or 19.5 % of the total protein.

After acidification to pH 3.15 with glacial acetic acid, a portion of this solution was gel filtered on a $1.5 \times 90$ cm. column of Sephadex G-50 (Superfine) equilibrated and eluted with 1 M acetic acid at 4-8 °C. The [N-formyl-Gly[1]A] human insulin peak (elution volume 87-95 ml) was pooled, aliquotted and lyophilized. This protein was judged to be quite clean by HPLC and amino acid analysis. The bioactivity of [N-formyl-Gly[1]-A] human insulin evaluated by radioreceptor assay was 17 % relative to a human insulin standard.

## Claims

1. A process for combining an A-chain of an insulin or an insulin analog and B-chain of an insulin or an insulin analog to produce an insulin or an insulin analog, which comprises reacting the S-sulfonated form of the A-chain, the S-sulfonated form of the B-chain, and a thiol reducing agent together in an aqueous medium under conditions which produce a mixture having (1) a pH of from 8 to 12, (2) a total protein concentration of from 0.1 to 50 milligrams per milliliter, and (3) an amount of thiol reducing agent which affords a total of from 0.4 to 2.5 —SH groups per each —SSO$_3^-$ group present in the total amount of A- and B-chain S-sulfonates, and allowing formation of insulin or an insulin analog to occur by maintaining the mixture at a temperature of from 0 °C to 25 °C and in an environment which provides a source of oxygen.

2. Process of claim 1, in which the A-chain S-sulfonate and the B-chain S-sulfonate each has the amino acid sequence represented by a naturally-occurring insulin.

3. Process of claim 2, in which the weight ratio of A-chain S-sulfonate to B-chain S-sulfonate is from 0.1 : 1 to 10 : 1.

4. Process of claim 3, in which the weight ratio A-chain S-sulfonate to B-chain S-sulfonate is from 1 : 1 to 3 : 1.

5. Process of any one of claims 1 to 4, in which the protein concentration is from 2 to 20 milligrams per milliliter.

6. Process of any one of claims 1 to 5, in which the pH of the reaction mixture is from 9.5 to 11.0.

7. Process of claim 6, in which the pH of the reaction mixture is from 10.4 to 10.6.

8. Process of any one of claims 1 to 7, in which the thiol reducing agent is present in an amount which affords a total of from 0.9 to 1.1 — SH group per each — SSO$_3$ group present in the total amount of A- and B-chain S-sulfonates.

9. Process of claim 8, in which the thiol reducing agent is dithiothreitol or dithioerythritol.

10. Process of any one of claims 1 to 4, in which the reaction mixture is maintained at a temperature of from 2 °C to 10 °C.

11. Process of any one of the preceding claims, in which the insulin that is produced is bovine, porcine, or human insulin.

12. Process of claim 11, in which the insulin that is produced is bovine insulin, the weight ratio of A-chain S-sulfonate to B-chain S-sulfonate is from 1.4 : 1.0 to 1.8 : 1.0, and the protein concentration is from 3 to 8 milligrams per milliliter.

13. Process of claim 11, in which the insulin that is produced is porcine insulin, the weight ratio of A-chain S-sulfonate to B-chain S-sulfonate is from 1.0 : 1.0 to 1.4 : 1.0, and the protein concentration is from 8 to 16 milligrams per milliliter.

14. Process of claim 11, in which the insulin that is produced is human insulin, the weight ratio of A-chain S-sulfonate to B-chain S-sulfonate is from 1.8 : 1.0 to 2.2 : 1.0, and the protein concentration is from 3 to 8 milligrams per milliliter.

## Ansprüche

1. Verfahren zur Herstellung eines Insulins oder Insulinanalogen durch Kombination einer A-Kette eines Insulins oder Insulinanalogen und einer B-Kette eines Insulins oder Insulinanalogen, dadurch gekennzeichnet, daß man die S-sulfonierte Form der A-Kette, die S-sulfonierte Form der B-Kette und ein Thiolreduktionsmittel zusammen in einem wäßrigen Medium unter Bedingungen umsetzt, unter denen ein Gemisch entsteht mit (1) einem pH-Wert von 8 bis 12, (2) einer gesamten Proteinkonzentration von 0,1 bis 50 mg/ml und (3) einer Menge an Thiolreduktionsmittel, die insgesamt 0,4 bis 2,5 Gruppen —SH pro Gruppe —$SSO_3^-$ in der Gesamtmenge an A- und B-Kette-S-Sulfonaten ergibt, und die Bildung von Insulin oder Insulinanalogen ablaufen läßt, indem man das Gemisch auf einer Temperatur von 0 °C bis 25 °C und in einer Umgebung, die für eine Sauerstoffquelle sorgt, hält.

2. Verfahren nach Anspruch 1, dadurch gekennzeichnet, daß das A-Kette-S-Sulfonat und das B-Kette-S-Sulfonat jeweils die Aminosäuresequenz eines natürlich vorkommenden Insulins hat.

3. Verfahren nach Anspruch 2, dadurch gekennzeichnet, daß das Gewichtsverhältnis von A-Kette-S-Sulfonat zu B-Kette-S-Sulfonat 0,1 : 1 bis 10 : 1 ausmacht.

4. Verfahren nach Anspruch 3, dadurch gekennzeichnet, daß das Gewichtsverhältnis von A-Kette-S-Sulfonat zu B-Kette-S-Sulfonat 1 : 1 bis 3 : 1 ausmacht.

5. Verfahren nach einem der Ansprüche 1 bis 4, dadurch gekennzeichnet, daß die Proteinkonzentration etwa 2 bis 20 mg/ml beträgt.

6. Verfahren nach einem der Ansprüche 1 bis 5, dadurch gekennzeichnet, daß das Reaktionsgemisch einen pH-Wert von 9,5 bis 11 hat.

7. Verfahren nach Anspruch 6, dadurch gekennzeichnet, daß das Reaktionsgemisch einen pH-Wert von 10,4 bis 10,6 hat.

8. Verfahren nach einem der Ansprüche 1 bis 7, dadurch gekennzeichnet, daß das Thiolreduktionsmittel in einer Menge vorhanden ist, die insgesamt 0,9 bis 1,1 —SH Gruppen pro —$SSO_3^-$ Gruppe in der Gesamtmenge aus A- und B-Kette-S-Sulfonaten ergibt.

9. Verfahren nach Anspruch 8, dadurch gekennzeichnet, daß man als Thiolreduktionsmittel Dithiothreit oder Dithioerythrit verwendet.

10. Verfahren nach einem der Ansprüche 1 bis 4, dadurch gekennzeichnet, daß man das Reaktionsgemisch auf einer Temperatur von 2 bis 10 °C hält.

11. Verfahren nach einem der vorhergehenden Ansprüche, dadurch gekennzeichnet, daß man als Insulin Rinderinsulin, Schweineinsulin oder Humaninsulin herstellt.

12. Verfahren nach Anspruch 11, dadurch gekennzeichnet, daß man als Insulin Rinderinsulin herstellt, das Gewichtsverhältnis von A-Kette-S-Sulfonat zu B-Kette-S-Sulfonat 1,4 : 1,0 bis 1,8 : 1,0 beträgt und die Proteinkonzentration bei 3 bis 8 mg/ml liegt.

13. Verfahren nach Anspruch 11, dadurch gekennzeichnet, daß man als Insulin Schweineinsulin herstellt, das Gewichtsverhältnis von A-Kette-S-Sulfonat zu B-Kette-S-Sulfonat 1,0 : 1,0 bis 1,4 : 1,0 beträgt und die Proteinkonzentration bei 8 bis 16 mg/ml liegt.

14. Verfahren nach Anspruch 11, dadurch gekennzeichnet, daß man als Insulin Humaninsulin herstellt, das Gewichtsverhältnis von A-Kette-S-Sulfonat zu B-Kette-S-Sulfonat 1,8 : 1,0 bis 2,2 : 1,0 beträgt und die Proteinkonzentration bei 3 bis 8 mg/ml liegt.

## Revendications

1. Procédé en vue de combiner une chaîne A et une chaîne B d'une insuline ou d'un analogue d'insuline pour produire une insuline ou un analogue d'insuline, caractérisé en ce qu'il consiste à faire réagir la forme S-sulfonée de la chaîne A, la forme S-sulfonée de la chaîne B et un agent réducteur à base d'un thiol ensemble dans un milieu aqueux et dans des conditions donnant un mélange ayant (1) un pH de 8 à 12, (2) une concentration totale en protéines de 0,1 à 50 mg par ml et (3) un agent réducteur à base d'un thiol en une quantité donnant, au total, 0,4 à 2,5 groupes —SH par groupe —$SSO_3^-$ présent dans la quantité totale des S-sulfonates des chaînes A et B, puis laisser se former une insuline ou un analogue d'insuline en maintenant le mélange à une température de 0 à 25 °C et dans un milieu ambiant fournissant une source d'oxygène.

2. Procédé suivant la revendication 1, caractérisé en ce que le S-sulfonate de la chaîne A et le S-sulfonate de la chaîne B ont chacun la séquence d'acides aminés représentée par une insuline naturelle.

3. Procédé suivant la revendication 2, caractérisé en ce que le rapport pondéral entre le S-sulfonate de la chaîne A et le S-sulfonate de la chaîne B est de 0,1 : 1 à 10 : 1.

4. Procédé suivant la revendication 3, caractérisé en ce que le rapport pondéral entre le S-sulfonate de la chaîne A et le S-sulfonate de la chaîne B est de 1 : 1 à 3 : 1.

5. Procédé suivant l'une quelconque des revendications 1 à 4, caractérisé en ce que la concentration en protéines se situe entre 2 et 20 mg par ml.

6. Procédé suivant l'une quelconque des revendications 1 à 5, caractérisé en ce que le pH du mélange réactionnel se situe entre 9,5 et 11.

7. Procédé suivant la revendication 6, caractérisé en ce que le pH du mélange réactionnel se situe entre 10,4 et 10,6.

8. Procédé suivant l'une quelconque des revendications 1 à 7, caractérisé en ce que l'agent réducteur à base d'un thiol est présent en une quantité donnant, au total, 0,9 à 1,1 groupe —SH par

**0 037 256**

groupe —$SSO_3^-$ présent dans la quantité totale des S-sulfonates des chaînes A et B.

9. Procédé suivant la revendication 8, caractérisé en ce que l'agent réducteur à base d'un thiol est le dithiothréitol ou le dithioérythritol.

10. Procédé suivant l'une quelconque des revendications 1 à 4, caractérisé en ce que le mélange réactionnel est maintenu à une température se situant entre 2 et 10 °C.

11. Procédé suivant l'une quelconque des revendications précédentes, caractérisé en ce que l'insuline produite est l'insuline bovine, l'insuline porcine ou l'insuline humaine.

12. Procédé suivant la revendication 11, caractérisé en ce que l'insuline produite est l'insuline bovine, le rapport pondéral entre le S-sulfonate de la chaîne A et le S-sulfonate de la chaîne B se situe entre 1,4 : 1 et 1,8 : 1, tandis que la concentration en protéine se situe entre 3 et 8 mg par ml.

13. Procédé suivant la revendication 11, caractérisé en ce que l'insuline produite est l'insuline porcine, le rapport pondéral entre le S-sulfonate de la chaîne A et le S-sulfonate de la chaîne B se situe entre 1 : 1 et 1,4 : 1, tandis que la concentration en protéine se situe entre 8 et 16 mg par ml.

14. Procédé suivant la revendication 11, caractérisé en ce que l'insuline produite est l'insuline humaine, le rapport pondéral entre le S-sulfonate de la chaîne A et le S-sulfonate de la chaîne B se situe entre 1,8 : 1 et 2,2 : 1, tandis que la concentration en protéine se situe entre 3 et 8 mg par ml.